**Europäisches Patentamt**

**European Patent Office**

**Office européen des brevets**

(19)

(11) Veröffentlichungsnummer: **0 308 966**
**A2**

(12) # EUROPÄISCHE PATENTANMELDUNG

(21) Anmeldenummer: **88115707.7**

(22) Anmeldetag: **23.09.88**

(51) Int. Cl.⁴: **G01N 11/16 , G01N 33/38 , B28C 5/08 , B28C 7/12**

(30) Priorität: **24.09.87 DE 3732231**

(43) Veröffentlichungstag der Anmeldung:
**29.03.89 Patentblatt  89/13**

(84) Benannte Vertragsstaaten:
**DE ES FR GB IT**

(71) Anmelder: **Hudelmaier, Ingrid**
**Sylvanerweg 19**
**D-7900 Ulm/Donau(DE)**

(72) Erfinder: **Waitzinger, Franz**
**Annastr. 13**
**D-7916 Nersingen(DE)**
Erfinder: **Hudelmaier, Gerhard, Dr.**
**Sylvanerweg 19**
**D-7900 Ulm/ Donau(DE)**

(74) Vertreter: **Patentanwälte Grünecker,**
**Kinkeldey, Stockmair & Partner**
**Maximilianstrasse 58**
**D-8000 München 22(DE)**

(54) **Verfahren zum Bestimmen der Konsistenz von Beton und zugehöriger Betonmischer.**

(57) Es sind Verfahren bekannt, bei denen die Konsistenz des zu mischenden Betons über die Leistungsaufnahme der in Drehbewegung versetzen Mischtrommel bestimmt wird. Hierbei wirken die Innenseiten der Mischtrommel als der Bewegung entgegengesetzte Widerstandsfläche, wobei sich jedoch die wirksame Widerstandsfläche abhängig von dem Füllgrad und der jeweiligen Lage der Mischtrommel zur Horizontalen ändert. Die Konsistenzmessung ist daher ungenau.

Das erfindungsgemäße Verfahren sieht daher vor, daß zum Bestimmen der Konsistenz des Betons während der gesamten Meßphase ein gegenüber dem Beton bewegter Staukörper in den Beton eingetaucht wird, dessen Strömungswiderstand gemessen wird. Der Staukörper setzt dem Beton stets eine definierte Widerstandfläche entgegen, so daß die Messungen weitgehend unabhängig von Störgrößen sind.

Das Verfahren findet Anwendung zum Einstellen der Konsistenz von Beton in sogenannten Fahrmischern.

FIG.1

Xerox Copy Centre

## Verfahren zum Bestimmen der Konsistenz von Beton und zugehöriger Betonmischer

Die Erfindung bezieht sich auf ein Verfahren zum Bestimmen der Konsistenz von Beton in einem Bewegungsmischer durch Messen eines Bewegungswiderstandes und auf einen zugehörigen Betonmischer mit einer Mischtrommel und einer Einrichtung zum Bestimmen der Konsistenz des Betons, die eine im Inneren der Trommel angeordnete und während der Meßphase zumindest teilweise mit dem Beton in Berührung kommende Widerstandsfläche aufweist.

Ein solches Verfahren und ein solcher Betonmischer sind aus der Praxis bekannt. Die Konsistenzbestimmung erfolgt über eine Messung der Leistungsaufnahme der Mischtrommel. Hierzu werden das Antriebsmoment oder auch der Hydraulikdruck eines die Mischtrommel antreibenden Hydromotors gemessen. Die Widerstandsfläche, die sich der Betonbewegung entgegensetzt, und die das Antriebsmoment der Mischtrommel wesentlich bestimmt, bilden die Innenfläche der Mischtrommel und in der Mischtrommel vorgesehene Einbauten, wie Mischerflügel u.dgl. Diese Widerstandsflächen sind während der Meßphase der Leistungsaufnahme der Mischtrommel nur zeitweilig mit dem Beton in Berührung. Die Größe der Widerstandsfläche hängt stark von dem Ladegrad der Mischtrommel ab. Hinzukommt, daß das Antriebsmoment für die Mischtrommel sehr stark davon abhängig ist, ob der Betonmischer auf einem ebenen Untergrund steht oder nicht. Insbesondere bei Fahrmischern kann nicht sichergestellt werden, daß diese vor Ort an der Baustelle immer auf einem ebenen Untergrund stehen. Da die Einstellung der Betonkonsistenz durch Vergleich des gemessenen Antriebsmomentes mit einem vorbestimmten Antriebsmoment erfolgt, ist aufgrund der vielen Störeinflüsse mit dem bekannten Verfahren und dem bekannten Betonmischer eine einwandfreie Konsistenzeinstellung nicht gezielt möglich, sondern eher vom Zufall abhängig.

Der Erfindung liegt daher die Aufgabe zugrunde, ein Verfahren und einen zugehörigen Betonmischer so auszubilden, daß sich die Konsistenz des Betons sicherer bestimmen und damit auch sicherer einstellen läßt.

Diese Aufgabe wird hinsichtlich des Verfahrens dadurch gelöst, daß man den Strömungswiderstand eines während der Meßphase in Beton eingetauchten und gegenüber dem Beton bewegten Staukörper mißt. Das hat den Vorteil, daß die dem Beton ausgesetzte Widerstandsfläche des Staukörpers während der gesamten Meßphase konstant bleibt. Diese Widerstandsfläche ist unabhängig von dem Füllgrad des Betonmischers und auch unabhängig davon, ob der Betonmischer auf einem ebenen oder schiefen Untergrund steht. Die Bestimmung der Konsistenz des in dem Betonmischer aufzubereitenden Betons ist daher im wesentlichen unabhängig von äußeren Störeinflüssen.

Zwar ist aus der DE-OS 31 13 785 eine Vorrichtung zur Bestimmung der Konsistenz von angemachtem Beton bekannt. Diese Vorrichtung benötigt einen Meßwerkrührer zur Erzeugung eines Betonstromes, wobei der Betonstrom auf ein Meßwiderlager gerichtet ist, dessen Verformung als Maß für die Konsistenz des Betons gemessen wird. Diese Vorrichtung ist verhältnismäßig aufwendig, da sie neben einem Meßwiderlager einen Meßwerkrührer und einen eigenen Antrieb erfordert. Bei dem erfindungsgemäßen Verfahren wird hingegen die Bewegung des Betons, die dieser aufgrund der Trommeldrehung oder anderer Bewegungseinrichtungen ohnehin ausführt, ausgenutzt, um die Konsistenz des Betons zu bestimmen.

Gemäß einer besonders bevorzugten Ausgestaltung des Verfahrens mißt man den durch eine Schwingbewegung des Stau körpers im Beton entstehenden Strömungswiderstand. Der Staukörper wird nun also nicht oder nicht nur passiv von Beton umströmt, sondern wird aktiv eine Strömung. Auf diese Weise wird die Messung des Strömungswiderstandes, der ja abhängig von der Zähigkeit und damit von der Konsistenz des Betons ist, im wesentlichen nur noch von der Art der Schwingbewegung des Staukörpers beeinflußt. Die Schwingbewegung des Staukörpers kann aber durch die Leistungsaufnahme des Schwingantriebs des Staukörpers gut kontrolliert werden.

Es ist günstig, wenn man den gemessenen Strömungswiderstand mit einem vorbestimmten, einer gewünschten Konsistenz zugeordneten Strömungswiderstand vergleicht und durch Zugabe von Flüssigkeit zu dem Beton an den vorbestimmten Strömungswiderstand angleicht. Auf diese Weise wird eine sichere Annäherung des Betons an die gewünschte Konsistenz erreicht.

Es ist besonders zweckmäßig, wenn man zur Konsistenzeinstellung des Betons nach und nach Flüssigkeit zugibt und nach einer Flüssigkeitszugabe den Beton so lange mischt, bis der gemessene Strömungswiderstand einen im wesentlichen konstanten Wert annimmt, bevor eine erneute Flüssigkeitszugabe erfolgt. So kann man sichergehen, daß der Beton bereits gut durchgemischt wurde, bevor der Beton durch eine erneute Flüssigkeitszugabe flüssiger eingestellt wird.

Da sich die Konsistenz des Betons und damit auch der zu messende Strömungswiderstand nicht unbedingt linear mit der Mischzeit ändert, kann die Aufbereitungszeit für den Beton in vorteilhafter Weise dadurch verkürzt werden, daß man nach einer Flüssigkeitszugabe fortlaufend die Änderung des gemessenen Strömungswiderstandes mißt und den Beton so lange mischt, bis der gemessene Wert der Änderung kleiner ist als ein vorgegebener Sollwert, bevor eine erneute Flüssigkeitszugabe erfolgt. Diese Vorgehensweise beschleunigt die Aufbereitung des Betons insbesondere zu Beginn, wenn der gemessene Strömungswiderstand noch weit oberhalb des vorbestimmten Strömungswiderstandes liegt.

Hinsichtlich des Betonmischers wird die Aufgabe erfindungsgemäß dadurch gelöst, daß die Widerstandsfläche auf einem zumindest während der Meßphase vollständig in den Beton eingetauchten Staukörper ausgebildet ist. Mit einem solchen Betonmischer läßt sich das erfindungsgemäße Verfahren besonders einfach ausführen.

Eine baulich besonders einfache Lösung kann bei Betonmischern mit drehbarer Mischtrommel dadurch vorgesehen sein, daß der Staukörper feststehend an der Innenseite der Mischtrommel angeordnet ist. Diese Anordnung des Staukörpers erlaubt statische Messungen der Betonkonsistenz. Der Beton wird durch die Drehung der Mischtrommel an dem Staukörper vorbeigeführt, so daß keine Bewegungseinrichtungen für den Staukörper vorzusehen sind. Mit einer solchen Anordnung des Staukörpers lassen sich insbesondere herkömmliche Betonmischer einfach nachrüsten.

Bei Betonmischern, die einen Vibrationskörper aufweisen, ist es günstig, wenn der Vibrationskörper selbst den Staukörper bildet. Solche Vibrationskörper sind beispielsweise in der DE-PS 32 01 162 beschrieben. Der Vorteil der Ausbildung dieser Vibrationskörper als Staukörper liegt insbesondere darin, daß die Strömung um den Vibrationskörper im wesentlichen durch die Schwingbewegung des Vibrationskörpers erzeugt wird und damit weitgehend unabhängig von äußeren Einflüssen ist. Der Einfluß der Trommeldrehung läßt sich meist vernachlässigen, wenn der Vibrationskörper im wesentlichen in der Mitte der Mischtrommel angeordnet ist.

Der Strömungswiderstand läßt sich bei einem hydraulisch angetrieben Vibrationskörper besonders leicht dadurch messen, daß in dem Hydraulikantrieb ein Druckaufnehmer angeordnet ist. Der Strömungswiderstand wird in diesem Fall also indirekt über die Leistungsaufnahme des Hydraulikantriebs des Vibrationskörpers gemessen.

Die Betonkonsistenz läßt sich besonders leicht und sicher einstellen, wenn die Einrichtung zum Bestimmen der Betonkonsistenz eine Steuerung umfaßt, die die mit dem Druckaufnehmer gemessenen Werte mit vorbestimmten Druckwerten vergleicht. Die Steuerung kann so ausgelegt sein, daß der vorbestimmte Druckwert mit einem Mittelwert oder einem Variationskoeffizienten verglichen wird. Die Messung kann auch über eine Integralauswertung erfolgen. Die Messungen können nach einer bestimmten Zeit, während des Mischens, nach einer bestimmten Anzahl von Trommelumdrehungen oder in einer bestimmten Trommelstellung vorgenommen werden. Zweckmäßigerweise wird der Zustand der Steuerungseinrichtung vor dem Start überprüft und als Korrekturfaktor für die Messung berücksichtigt.

Es ist zweckmäßig, wenn die Steuerung ein Steuersignal erzeugt, wenn der mit dem Druckaufnehmer gemessene Wert über einen vorbestimmten Zeitraum im wesentlichen konstant bleibt, oder die Änderung der Druckwerte ein vorbestimmtes Maß unterschreitet. Mit dem ausgesendeten Steuersignal kann dann ein Wasserventil geschaltet werden, so daß eine weitere Flüssigkeitszufuhr erfolgt, falls der zuvor mit dem Druckaufnehmer gemessene Wert noch oberhalb des vorbestimmten Druckwertes liegt.

Besonders günstig ist es, wenn die Steuerung ein Abschaltsignal erzeugt, wenn der mit dem Druckaufnehmer gemessene Wert über einen vorbestimmten Zeitraum etwa dem vorbestimmten Druckwert entspricht. Dieses Abschaltsignal gibt an, daß nun der Beton die gewünschte Konsistenz auf weist. Mit Hilfe dieser Steuerung läßt sich die Konsistenz des Betons sicher und vollautomatisch auf den gewünschten Wert einstellen.

Im folgenden wird die Erfindung anhand einer Zeichnung näher erläutert. Es zeigen:

Fig. 1 einen erfindungsgemäßen Betonmischer gemäß einer ersten Ausführungsform in einer teilweise aufgeschnittenen Seitenansicht,

Fig. 2 den Betonmischer aus Fig. 1 in einer schematischen Seitenansicht, im gefüllten Zustand,

Fig. 3 in einer ähnlichen Ansicht, wie Fig. 2 ein zweites Ausführungsbeispiel eines erfindungsgemäßen Betonmischers, und

Fig. 4 ein Detail aus der Fig. 3 im Schnitt entlang der Linie IV-IV und

Fig. 5 ein Diagramm, das den Steuerungsablauf zum Einstellen der Betonkonsistenz in dem Betonmischer darstellt.

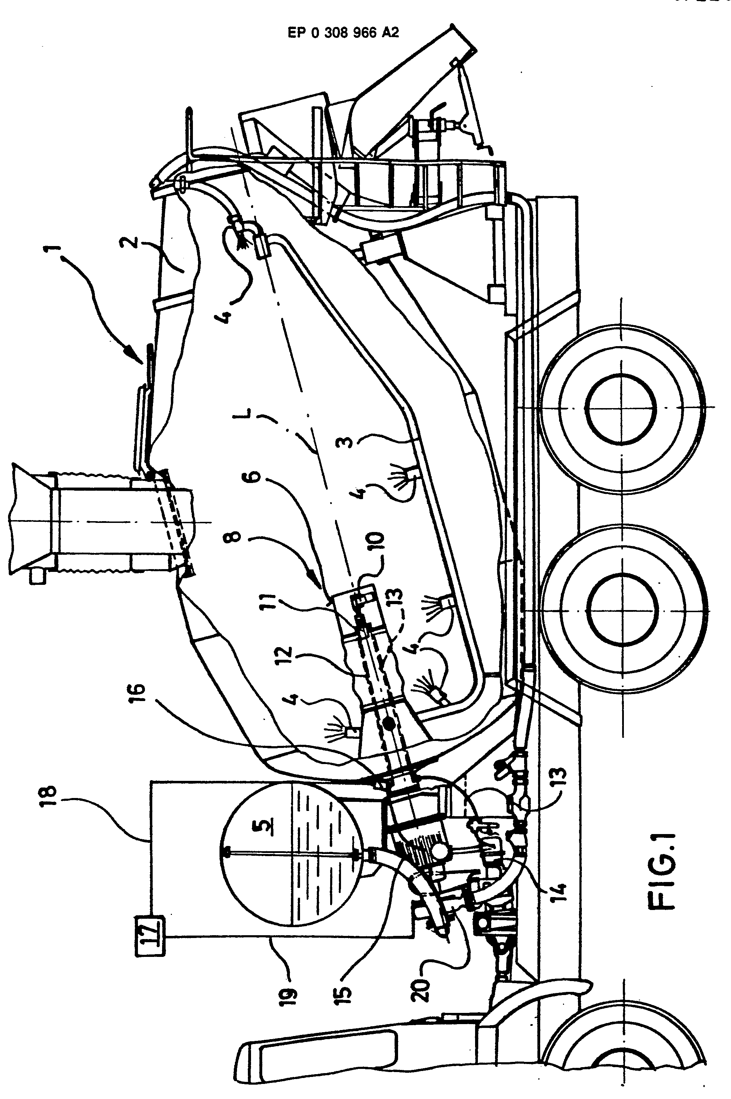

In den Fig. 1 bis 3 ist ein Betonmischer, genauer ein sogenannter Fahrmischer 1, in einer teilweise aufgeschnittenen Seitenansicht dargestellt. Dieser Fahrmischer 1, der auf einem Fahrgestell eines Lastkraftwagens aufgesetzt ist, umfaßt im wesentlichen eine drehbare Mischtrommel 2 und eine im Inneren angeordnete Wasserzuführleitung 3 mit Austrittsdüsen 4, aus denen das Anmachwasser aus einem Wassertank 5 über die Wasserzuführleitung 3 ins Innere der Mischtrommel 2 austreten kann.

Ferner umfaßt der Fahrmischer 1 eine Einrichtung zum Bestimmen der Konsistenz des im Inneren der Mischtrommel 2 zu mischenden Betons. Sämtlichen Ausführungsbeispielen der Erfindung ist gemein, daß diese Einrichtung eine zumindest während der Meßphase mit dem Beton in Berührung kommende Widerstandsfläche 6 bzw. 7 aufweist. Die Widerstandsflächen 6 bzw. 7 sind auf einem Staukörper 8 bzw. 9 so ausgebildet, daß sie während der Meßphase vollständig in dem Beton eingetaucht sind.

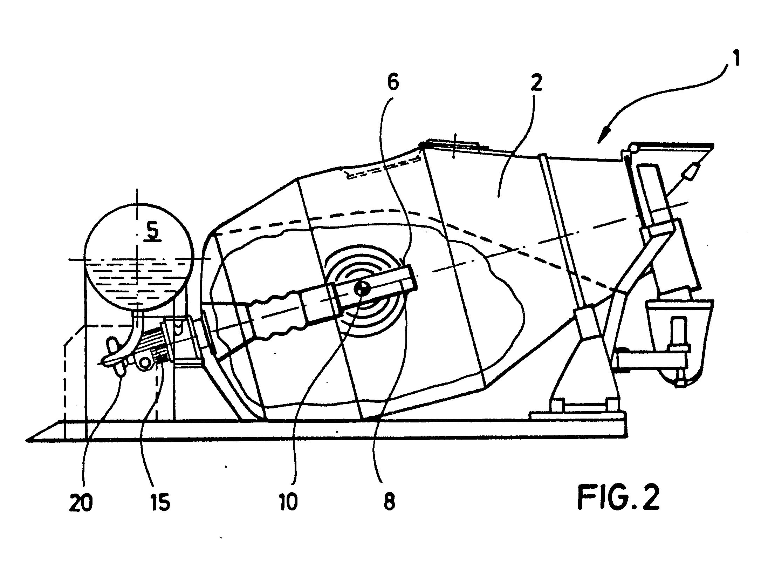

Bei dem ersten Ausführungsbeispiel, das in den Fig. 1 und 2 dargestellt ist, ist der Staukörper 8 als Vibrationskörper ausgebildet. Ein solcher Vibrationskörper ist bereits in der DE-PS 32 01 162 beschrieben. Der Vibrationskörper 8, der bei dem hier beschriebenen Ausführungsbeispiel Verwendung findet, ist schematisch besonders gut aus Fig. 1 erkennbar. Es handelt sich um einen Zylinder, der auf der Längsmittenachse der Mischtrommel 2 angeordnet ist. In dem Zylinder des Vibrationskörpers 8 ist ein Unwuchterreger 10 in Form einer exzentrisch angeordneten und drehbaren Schwungmasse vorgesehen. Der Unwuchterreger ist über eine nicht näher bezeichnete Welle mit einem hydraulisch betriebenen Zahnradmotor 11 gekuppelt. Von dem Zahnradmotor 11 erstrecken sich Hydraulikleitungen 12 und 13 durch das vordere Ende der Mischtrommel 2 hindurch zu einer Hydraulikpumpe 14. Zwischen die Hydraulikpumpe 14 und den Zahnradmotor 11 ist noch der hydraulische Drehantrieb 15 für die Mischtrommel 2 zwischengeschaltet, der jedoch herkömmlicher Bauart ist und daher nicht näher beschrieben wird.

An mindestens einer der Hydraulikleitungen 12 bzw. 13 ist ein Druckaufnehmer 16 angeordnet, der über eine Steuerleitung 18 mit einer Steuereinrichtung 17 verbunden ist. Die Steuereinrichtung 17 ist über eine weitere Steuerleitung 19 mit einer Speisepumpe 20 für die Zuführung des Anmachwassers aus dem Wassertank 5 in das Innere der Mischtrommel 2 verbunden. Alternativ kann die Steuereinrichtung 17 auch an ein hier nicht dargestelltes Abschaltventil in der Wasserzuführleitung 3 angeschlossen sein.

Die Steuereinrichtung 17 ist so ausgebildet, daß sie die mit dem Druckaufnehmer 16 gemessenen Werte mit einem vorbestimmten Sollwert vergleicht. Der Sollwert kann mittels eines Programms vorbestimmt werden, welches sich auf die Konsistenz auswirkende Einflußgrößen, wie Zusammensetzung des Betons, Zustand der Zuschläge und des Zements berücksichtigt.

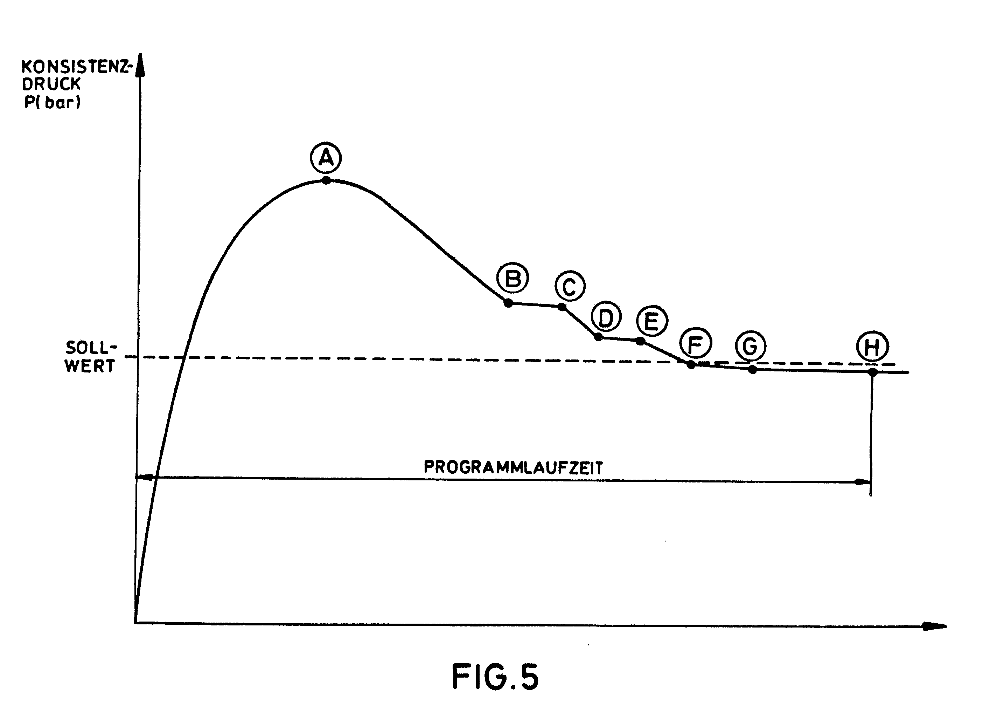

Im folgenden wird das erfindungsgemäße Verfahren am Beispiel eines Fahrmischers gemäß den Fig. 1 und 2 und anhand des in Fig. 5 gezeigten Programmablaufs näher erläutert.

Nach Befüllen der Mischtrommel 2 wird diese durch den Drehantrieb 15 in Rotation um ihre Längsachse L versetzt. Dies geschieht dadurch, daß in den Drehantrieb 15 durch die Hydraulikpumpe 14 und über die zugehörigen Hydraulikleitungen ein Druckfluid gepumpt wird. Es wird dann auch über die Hydraulikpumpe 14 und die Hydraulikleitungen 12 und 13 der Zahnradmotor 11 des Unwuchterregers 10 in Drehung versetzt, so daß der Vibrationskörper 8 in Exzenterschwingungen versetzt wird. Dadurch bewegt sich die Mantelfläche 6 des Vibrationskörpers, die vollständig in die Betonmischung eingetaucht ist, relativ zu dieser Mischung. Der Mantelfläche 6 des Vibrationskörpers 8 wird durch den in der Mischtrommel 2 befindlichen Beton ein Widerstand entgegengesetzt, der abhängig ist von der Konsistenz des Betons. Der Schwingbewegung des Vibrationskörpers 8 wird bei weitgehend trockener Betonmischung ein starker Widerstand entgegengebracht. Durch diese Schwingbewegungen verdichtet sich die Betonmischung, so daß der Druck in den Hydraulikleitungen 12 und 13, der durch den Druckaufnehmer 16 gemessen wird, in verhältnismäßig kurzer Zeit auf einen maximalen Druckwert A ansteigt. Bereits während des Druckanstieges schaltet die Steuereinrichtung 17 die Speisepumpe 20 an, oder öffnet die Wasserzuführleitung 3 über ein hier nicht dargestelltes Ventil, so daß Anmachwasser aus dem Wassertank 5 über die Wasserzuführleitung 3 und die Austrittsdüsen 4 der Betonmischung zugeführt wird. Nachdem der von dem Druckaufnehmer 16 angezeigte maximale Druck, der auch der maximalen Leitungsaufnahme des Vibrationskörpers 8 entspricht, erreicht ist, bewirkt die Wasserzugabe, daß das Betongemisch geschmeidiger wird, so daß der mit dem Druckaufnehmer 16 gemessene Druck hinter der Stelle A abfällt. Mit Abstand oberhalb des vorbestimmten Drucksollwertes, der der gewünschten Konsistenz des Betons entspricht, wird die Wasserzugabe gestoppt (Stelle B). Die minimale Menge Anmachwasser, die auf jeden Fall benötigt wird, ist nun vollständig in die Mischtrommel 2 eingeführt worden. Der Vibrationskörper, bzw. der darin eingebaute Unwuchterreger 10, wird so lange weiterbetrieben, bis der im Druckaufnehmer 16 gemessene Druck über einen bestimmten Zeitraum (B-C) im wesentlichen konstant geblieben ist. Wie aus dem Ablaufdiagramm ersichtlich ist, ändert sich der Druck in dem Zeitabschnitt B-C nur noch geringfügig, was bereits für eine gute Durchmischung durch den Vibrationskörper 8 spricht.

An der Stelle C ist der durch den Druckaufnehmer 16 gemessene Druck noch deutlich höher als der Sollwert, so daß eine weitere Wasserzugabe erfolgt. Diese Wassermenge ist deutlich kleiner als die bereits zugeführte Mindestwassermenge. Wieder oberhalb des Sollwertes wird an der Stelle D die Wasserzugabe gestoppt, wonach der mit dem Druckaufnehmer 16 gemessene Druckabfall wieder sichtbar kleiner wird. Der gemessene Druck bleibt bis zur Stelle E nahezu konstant und liegt immer noch oberhalb des Sollwertes. An der Stelle E erfolgt daher eine erneute Zugabe an Anmachwasser, wobei die Menge hier wieder geringer ist als bei dem vorigen Schritt. Sobald an der Stelle F der mit dem Druckaufnehmer 16 gemessene Druck dem Drucksollwert entspricht, wird die Wasserzufuhr gänzlich abgestellt. Bis zur Stelle G hat der gemessene Druckwert einen konstanten Wert erreicht, woraufhin der Vibrationskörper 8 noch bis zur Stelle H weiterbetrieben wird, um die Homogenisierung des Betons noch weiter zu verbessern. An der Stelle H liegt in der Mischtrommel 2 Beton in der gewünschten Konsistenz vor.

Während dieses Steuerprogramms befindet sich der Vibrationskörper die ganze Zeit eingebettet im Beton, so daß die dem Beton ausgesetzte wirksame Widerstandsfläche 6, also die Mantelfläche des Vibrationskörpers 8, während der gesamten Meßphase keinen Änderungen unterworfen ist. Die Einstellung der Konsistenz kann daher weitgehend unabhängig von äußeren Einflüssen genau vorgenommen werden, wobei es insbesondere nicht stört, ob die Charge in der Mischtrommel 2 etwas größer oder etwas kleiner ist, oder wenn der Fahrmischer 1 auf unebenem Untergrund steht.

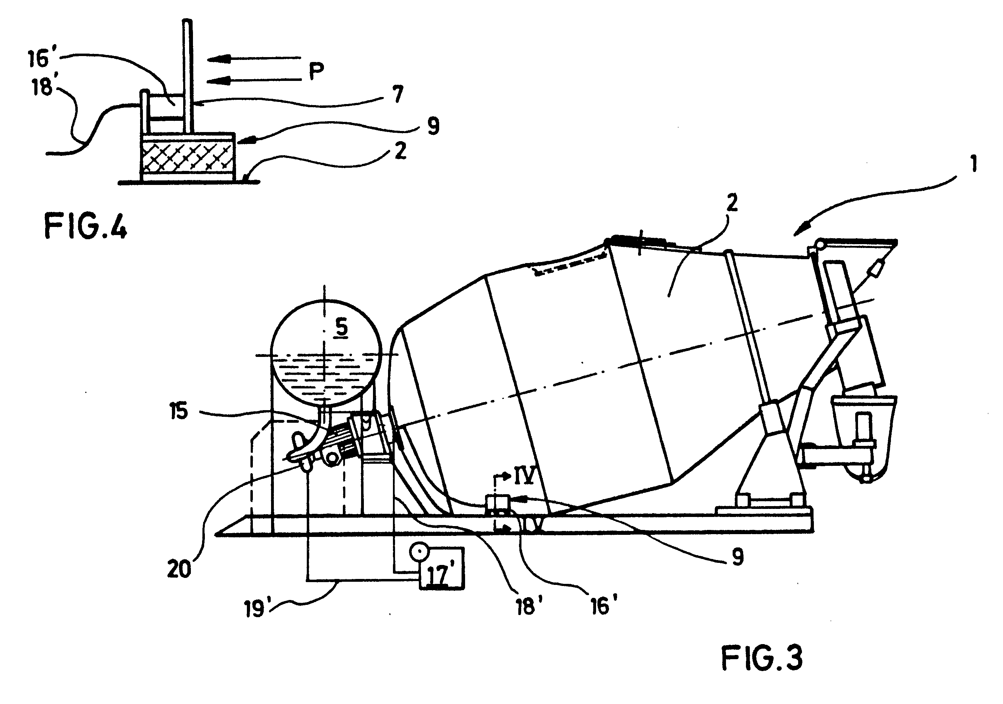

In den Fig. 3 und 4 ist ein weiteres Ausführungsbeispiel eines erfindungsgemäßen Betonmischers dargestellt, das in seiner Wirkungsweise prinzipiell dem oben beschriebenen Ausführungsbeispiel entspricht.

Für gleiche und ähnliche Bauteile werden daher identische Bezugszeichen verwendet; es werden nur die Unterschiede zu oben beschriebenem Ausführungsbeispiel erläutert.

Im Unterschied zum ersten Ausführungsbeispiel ist die Widerstandsfläche 7 an einem Staukörper 9 vorgesehen, der ortsfest im Inneren der Mischtrommel 2 angeordnet ist, sich also mit der Mischtrommel 2 mitdreht. Wie aus Fig. 4 ersichtlich ist, wird die Widerstandsfläche 7, die als Stauplatte bestimmter Größe ausgebildet ist, in Pfeilrichtung P von dem in der Mischtrommel 2 befindlichen Beton umströmt, d.h. die Stauplatte 7 wird entsprechend Fig. 4 von rechts mit Druck beaufschlagt.

Auf der Rückseite der Stauplatte 7 ist ein Druckaufnehmer 16' angeordnet, der mit einer Steuerleitung 18' an eine Steuereinrichtung 17' angeschlossen ist. Die Speisepumpe 20 des Betonmischers ist über eine Steuerleitung 19' mit der Steuereinrichtung 17' verbunden. Der Druckaufnehmer 16' kann beispielsweise als Piezoelement ausgebildet sein.

Wenn sich die Mischtrommel 2 um ihre Längsmittenachse L dreht, wird die Staufläche 7 des Staukörpers 9 in Richtung der Pfeile P mit Druck beaufschlagt, da sie dem Beton einen gewissen Strömungswiderstand entgegensetzt. Die Höhe des durch den Druckaufnehmer 16' gemessenen Drucks ist ein Maß für den Strömungswiderstand und damit zugleich ein Maß für die Viskosität des Betons und sogleich dessen Konsistenz. Dadurch, daß sich die Staufläche 7 an dem Staukörper 9 mit der Mischertrommel dreht, kann es je nach Anordnung des Staukörpers 9 an der Innenwand der Mischtrommel 2 vorkommen, daß der Staukörper 9 in den Beton eintaucht oder aus diesem heraustritt. Das bedeutet, daß der Druckaufnehmer 16' bei jeder Umdrehung der Mischtrommel 2 einen plötzlichen Druckabfall und einen plötzlichen Druckanstieg registriert. Der plötzliche Druckanstieg, wie auch der plötzliche Druckabfall kennzeichnen Beginn und Ende der Meßphase, so daß während der Meßphase die Widerstandsfläche 7 des Staukörpers 9 stets vollständig in den Beton eingetaucht ist. Durch diese Auslegung der Steuereinrichtung 17' läßt sich die Einstellung der gewünschten Konsistenz weitgehend unabhängig vom Füllgrad der Mischtrommel 2 bestimmen.

Abgesehen davon, daß bei dem zweiten Ausführungsbeispiel die Meßphase sich nur über einen Teil einer Trommelumdrehung erstreckt, unterscheidet sich das Verfahren zur Einstellung der Konsistenz des Betons gegenüber dem ersten Ausführungsbeispiel nicht. Die Steuereinrichtung 17' wird ebenso nach dem in Fig. 5 gezeigten Programmablauf betrieben.

Es ist auch möglich, die Widerstandsfläche so anzubringen, daß sie gegenüber dem Fahrgestell des Fahrmischers 1 ortsfest angeordnet ist und sich mit der Mischtrommel nicht mitdreht, so daß die Widerstandsfläche während einer vollständigen Trommeldrehung im Beton eingetaucht ist.

Um Einflüsse von Druckschwankungen, die durch Trockeneinschlüsse hervorgerufen werden können, zu vermeiden, kann die Steuereinrichtung so ausgelegt sein, daß die Steuerbefehle nur anhand von gemittelten Druckwerten, Variationskoeffizienten oder einer Integralauswertung erzeugt werden.

Die Druckmessungen werden bei den hier dargestellten Ausführungsbeispielen vorzugsweise jeweils bei konstanter Trommeldrehzahl bzw. gleichbleibender Schwingungsfrequenz des Vibrationskörpers durchgeführt. Im Falle, daß die Widerstandsfläche jedoch wie beim ersten Ausführungsbeispiel auf dem Vibrationskörper angeordnet ist, spielt der Einfluß der Trommeldrehzahl auf den durch die Widerstandsfläche ausgeübten Strömungswiderstand nur eine untergeordnete Rolle und kann daher je nach Ausbildung des Vibrationskörpers unberücksichtigt bleiben.

## Ansprüche

1. Verfahren zum Bestimmen der Konsistenz von Beton in einem Bewegungsmischer durch Messen eines Bewegungswiderstandes, **dadurch gekennzeichnet,** daß man den Strömungswiderstand eines während der Meßphase in den Beton eingetauchten und gegenüber dem Beton bewegten Staukörpers mißt.

2. Verfahren nach Anspruch 1, **dadurch gekennzeichnet,** daß man den durch eine Schwingbewegung des Staukörpers im Beton entstehenden Strömungswiderstand mißt.

3. Verfahren nach Anspruch 1 oder 2, **dadurch gekennzeichnet,** daß man den gemessenen Strömungswiderstand mit einem vorbestimmten, einer gewünschten Konsistenz zugeordneten Strömungswiderstand vergleicht und durch Zugabe von Flüssigkeit zu dem Beton an den vorbestimmten Strömungswiderstand angleicht.

4. Verfahren nach einem der Ansprüche 1 bis 3, **dadurch gekennzeichnet,** daß man zur Konsistenzeinstellung des Betons nach und nach Flüssigkeit zugibt und nach einer Flüssigkeitszugabe den Beton so lange mischt, bis der gemessene Strömungswiderstand einen im wesentlichen konstanten Wert annimmt, bevor eine erneute Flüssigkeitszugabe erfolgt.

5. Verfahren nach einem der Ansprüche 1 bis 4, **dadurch gekennzeichnet,** daß man nach einer Flüssigkeitszugabe fortlaufend die Änderungen des gemessenen Strömungswiderstandes mißt und den Beton so lange mischt, bis der gemessene Wert der Änderung kleiner ist als ein vorgegebener Soll-Wert, bevor eine erneute Flüssigkeitszugabe erfolgt.

6. Betonmischer zum Durchführen des Verfahrens nach einem der Ansprüche 1 bis 5, mit einer Mischtrommel (2) und einer Einrichtung zum bestimmen der Konsistenz des Betons, die eine im Inneren der Trommel (2) angeordnete und während der Meßphase zumindest teilweise mit dem Beton in Berührung kommende Widerstandsfläche (6, 7) aufweist, **dadurch gekennzeichnet,** daß die Widerstandsfläche (6, 7) auf einem zumindest während der Meßphase vollständig in den Beton eingetauchten Staukörper (8, 9) ausgebildet ist.

7. Betonmischer nach Anspruch 6, mit drehbarer Mischtrommel (2), **dadurch gekennzeichnet,** daß der Staukörper (9) feststehend an der Innenseite der Mischtrommel (2) angeordnet ist.

8. Betonmischer nach Anspruch 6 oder 7, mit einem Vibrationskörper (8), **dadurch gekennzeichnet,** daß der Vibrationskörper (8) den Staukörper bildet.

9. Betonmischer nach einem der Ansprüche 6 bis 8, mit einem hydraulisch angetriebenen Vibrationskörper (8), **dadurch gekennzeichnet,** daß in dem Hydraulikantrieb (11, 12, 13, 14) ein Druckaufnehmer (16) angeordnet ist.

10. Betonmischer nach einem der Ansprüche 6 bis 9, **dadurch gekennzeichnet,** daß die Einrichtung zum Bestimmen der Betonkonsistenz eine Steuereinrichtung (17) umfaßt, die die mit dem Druckaufnehmer (16, 16') gemessenen Werte mit vorbestimmten Druckwerten vergleicht.

11. Betonmischer nach einem der Ansprüche 6 bis 10, **dadurch gekennzeichnet,** daß die Steuerungseinrichtung (17, 17') ein Steuersignal erzeugt, wenn der mit dem Druckaufnehmer (16, 16') gemessene Wert über einen vorbestimmten Zeitraum (B-C, D-E) im wesentlichen konstant bleibt.

12. Betonmischer nach einem der Ansprüche 6 bis 11, **dadurch gekennzeichnet,** daß die Steuerungseinrichtung (17, 17') ein Abschaltsignal erzeugt, wenn der mit dem Druckaufnehmer (16, 16') gemessene Wert über einen vorbestimmten Zeitraum (G-H) etwa dem vorbestimmten Druckwert entspricht.

FIG.1

EP 0 308 966 A2

FIG.2

EP 0 308 966 A2

P 21 228

FIG.4

FIG.3

EP 0 308 966 A2

P 21228

**FIG.5**

Axis label (vertical): KONSISTENZ-DRUCK P(bar)

SOLL-WERT

PROGRAMMLAUFZEIT

A B C D E F G H

EP 0 308 966 A2